# EUROPEAN PATENT APPLICATION

(11) **EP 2 527 833 A1**
(43) Date of publication of application: **28.11.2012**
(21) Application number: 12166245.6
(22) Date of filing: 30.04.2012
(51) Int. Cl.: G01N 33/00, G01N 27/414, G01N 27/27, G01N 27/22

(54) **A device comprising a gas sensor sensitive to the presence of a specific gas, method of manufacturing a gas sensor sensitive to the presence of a specific gas for use in the device and use of the device**

(30) Priority: 28.04.2011 US 201161480306 P
(71) Applicant: Stichting IMEC Nederland, 5656 AE Eindhoven (NL)
(72) Inventor: Kochupurackal, Jinesh, 641664 Singapore (SG); Offermans, Peter, 5611 SZ Eindhoven (NL); Blauw, Michiel, 4818 RL Breda (NL); Crego Calama, Mercedes, 5663 PK Geldrop-Mierlo (NL); Brongersma, Sywert, 5644 GK Eindhoven (NL)
(74) Representative: Duyver, Jurgen Martha Herman

(57) **Abstract**

A device (100) comprising a gas sensor sensitive to the presence of a specific gas, the gas sensor comprising: a first segment (102) made of a dielectric material; and a second segment (104) made of a semiconducting material; wherein: the first segment (102) has a first surface (108) that is exposed to an environment of the gas sensor; the first segment (102) is located between the environment and the second segment (104); the first segment (102) has a first thickness; the second segment (104) has a second thickness (112); wherein the first thickness is selected such that upon diffusion of a gas molecule of the specific gas into the first segment (102), a dipole of the molecule of the specific gas detectably influences a bending of an energy-band structure of the semiconducting material of the second segment (104); and wherein the second thickness is in the order of the Debye length of the semiconducting material or smaller than the Debye length of the semiconducting material.

## Description

### Field of the disclosure

The disclosed technology relates to a device comprising a gas sensor that is sensitive to the presence of a specific, preferably non-redox, gas.

The disclosure also relates to methods of fabricating such gas sensors and use of the device according to the disclosure.

### Description of the related technology

A gas sensor is a sensor device that is operative to recognize the presence of a specific gas in the sensor's liquid or gaseous environment and to generate a sensor signal in dependence on this recognition. Typically, gas recognition relies on adsorption, chemical reactions or electrochemical reactions between the specific gas and a solid included in the gas sensor.

Direct electrical detection of a non-redox gas, such as for example carbon dioxide (CO₂) and/or Nitrogen dioxide (NO₂), is quite challenging because a non-redox gas is a gas that is chemically relatively inert, preferably under standard conditions, such as for example 273.15 K and 101.325 kPa, and more preferably does not readily undergoes redox reactions under those standard conditions, unless under specific conditions such as for example in the presence of a specific catalyst and under high temperature conditions as for example discussed below, and does therefore not react readily with the surface of most conductive materials, especially at the standard conditions or at ambient temperature, such as room temperature.

It is known that CO₂ can be detected using thin films or nanowires of materials such as tin dioxide (SnO₂), with lanthanum (La) as a catalyst to reduce CO₂ into the redox gas carbon monoxide (CO). A redox gas can for example be detected using a device with a metal-oxide field-effect transistor (MOxFET) configuration. However, for this catalytic action to occur there is a need for heating the device to a relatively high temperature (e.g. about 300°C).

In order to maintain a MOxFET-based gas sensor at such an elevated temperature, a heater is used with the gas sensor, e.g., physically integrated with the gas sensor. The heater contributes heavily to the total power consumption of the gas sensor in operational use. The relatively high power consumption is especially a disadvantage in wireless autonomous applications of the gas sensor, that is, in applications wherein the gas sensor is equipped with its own power supply and with a radio-frequency transmitter for wireless communication with a remote receiver. For wireless autonomous applications, devices operating at low power budgets are preferred so as to keep functioning autonomously for long periods of time while remaining powered by means of miniaturized or integrated low-power batteries. For these applications, the power budget may be less than, for example, 10⁻⁴ W. In contrast, devices that operate at elevated temperatures (e.g., higher than 100°C) typically consume in the order of 10⁻³ W of heating power.

### Summary of certain inventive aspects

The current disclosure relates to solid-state gas sensors for sensing non-redox gases wherein the sensors can operate at ambient temperature, e.g. room temperature. It is an advantage that the need for heating of the sensors during their operation can be avoided and that their power consumption can be low.

A gas sensor sensitive to the presence of a specific gas according to the current disclosure comprises a first segment, for example a first layer, made of a dielectric material, and a second segment, for example a second layer, made of a semiconducting material. The first segment has a first surface that is exposed to an environment of the gas sensor and, preferably a second surface oriented towards the second segment. The first segment is located between the environment and the second segment. The first segment has a first thickness, preferably measured between the first surface and the second surface. The second segment has a second thickness, the second thickness being in the order of the Debye length of the semiconducting material or smaller than the Debye length of the semiconducting material, for example a thickness in a range of sub-nanometer to about 10 nm, e.g. about 5 nm. The first thickness is selected such that upon diffusion of a gas molecule of the specific gas into the first segment, a dipole of the molecule of the specific gas can detectably influence a bending of an energy-band structure of the semiconducting material of the second segment.

According to preferred embodiments of the device, the gas sensor comprises a third segment of a further dielectric material, wherein the second segment is sandwiched between the first segment and the third segment.

According to preferred embodiments of the device, the gas sensor further comprises a first electrode in electrical contact with the second segment at a first end of the second segment and a second electrode in electrical contact with the second segment at a second end of the second segment, and the second segment forms a conduction channel for an electric current, preferably between the first electrode and the second electrode. The bending of the energy-band structure of the semiconducting material of the second segment upon diffusion of a gas molecule of the specific gas into the first segment can be detected by measuring an electrical current through the conduction channel upon applying a voltage difference between the first electrode and the second electrode, preferably by measuring a difference in the measured electrical current through the conduction channel.

According to preferred embodiments of the device, the gas sensor comprises a back-gate for receiving a control voltage, e.g. for controlling a sensitivity of the gas sensor. In this embodiment, the band-bending of the energy-band structure of the semiconducting material can be controlled using a back-gate voltage (control voltage).

According to preferred embodiments of the device, the gas sensor may include a plurality of sensor elements of assemblies of the first and the second segment. Different ones of the plurality of sensor elements may have different semiconducting materials in their respective second segments and/or they may have different dielectric materials in their respective first segments, so as to have a varying sensitivity per individual one of the plurality of sensor elements.

According to preferred embodiments of the device, the second segment has a length substantially larger than the second thickness, and the first segment encloses the second segment over the length of the second segment.

According to preferred embodiments of the device, the second segment is formed as at least one nanowire, for example a nanowire or a plurality of nanowires. If the device comprises a plurality of nanowires, the nanowires may be composed of the same semiconducting material or a respective one of the nanowires may be composed of a respective one of different semiconducting materials. Similarly, each respective one of the different nanowires may be enclosed by a respective first segment of a respective one of different dielectric materials so as to provide multiple sensing elements in the gas sensor of different sensitivity.

The disclosure also relates to a method of manufacturing a gas sensor sensitive to the presence of a specific gas for use in the device according to the current disclosure. The gas sensor comprises a first segment, preferably first layer, made of a dielectric material, and a second segment, preferably second layer, made of a semiconducting material. The first segment has a first surface that is provided to be exposed or is exposed to an environment of the gas sensor and a second surface oriented towards the second segment. The first segment is located between the environment and the second segment. The first segment has a first thickness, measured between the first surface and the second surface. The second segment has a second thickness, the second thickness being in the order of the Debye length of the semiconducting material or smaller than the Debye length of the semiconducting material. The first thickness is selected such that upon diffusion of a gas molecule of the specific gas into the first segment, a dipole of the molecule of the specific gas can detectably influence a bending of an energy-band structure of the semiconducting material of the second segment. The method comprises forming the first segment by depositing the dielectric material on the second segment. The depositing preferably comprises one of: an atomic layer deposition (ALD) method and a chemical vapor deposition (CVD) method. The use of ALD or CVD enables to suitably control the depositing conditions of the dielectric material so as to be able to tune a density of charges in the dielectric material of the first segment.

The density of the charges (e.g. fixed oxide charges, oxide trapped charges) in the dielectric material can be tuned by controlling the conditions under which the dielectric material is being deposited. For example, the density of the charges depends on, among other things, the deposition temperature, the choice of precursors, the presence of dopants, the chemical compound or the chemical compounds making up the dielectric material, etc. The term "precursors" refers here to the chemical reactants that are used to form the first segment of dielectric material. For example, in case the dielectric material is La₂O₃, the precursors can be La(thd)₃ ('thd" stands for 2,2,6,6-tetramethyl-3,5-heptanedionate) and oxygen. The precursors and the deposition conditions of the dielectric layer (first segment) are chosen so as to give rise to a stable density of electric charges in the dielectric material, especially with regard to the oxide trapped charges, the interface charges and the fixed oxide charges. For example, when aluminum oxide (Al₂O₃) is deposited by means of ALD using trimethyl aluminum and water as precursors, loosely bound -OH groups on AlₓO_{y}-molecules are found to act as oxide trap charges in the dielectric layer. The density of trapped charges can be estimated on the basis of capacitance-voltage measurements.

The present disclosure also relates to a use of the device according to the present disclosure for sensing the presence of a specific gas in the environment of the gas sensor, wherein the first surface of the first segment is exposed to the environment of the gas sensor; wherein upon diffusion of a gas molecule of the specific gas into the first segment, a dipole of the molecule of the specific gas detectably influences a bending of the energy-band structure of the semiconducting material of the second segment.

The operation of a gas sensor according to one aspect is not based on a chemical binding of a target species to the dielectric material. Instead, gas molecules diffusing into the dielectric layer (first segment) interact with charges in the dielectric layer and affect the effective charge density or the charge distribution in the dielectric layer, thus inducing mirror charges in the semiconductor layer (second segment) and changing band bending in the semiconductor layer. The major interaction between the charges in the dielectric layer of a device according to one inventive aspect and the gas molecules may be dipole interactions, which screen the electric field of the charges. This causes a bending of the energy-band-structure of the semiconducting material of the second segment. The thickness of the second segment is selected such that the electric properties of the semiconducting second segment are largely determined by the band-bending. Any change in the band-bending then gives rise to an effect that can be electrically measured. Preferably the thickness of the second segment is in the order of the Debye length of the semiconducting material or smaller than the Debye length of the semiconducting material.

According to preferred embodiments of the current disclosure, the specific gas is a non-redox gas.

According to preferred embodiments of the current disclosure, the bending of the energy-band structure of the semiconductor material of the second segment is detected by measuring a difference in an electrical current through the conduction channel upon applying a voltage difference between the first electrode and the second electrode.

According to preferred embodiments of the current disclosure, the semiconducting material of the second segment comprises silicon (Si), indium arsenide (InAs), indium phosphide (InP), gallium arsenide (GaAs), zinc oxide (ZnO₂), tungsten trioxide (WO₃ and/or tin dioxide (SnO₂).

According to preferred embodiments of the current disclosure, the dielectric material of the first segment comprises lanthanum oxide (La₂O₃), titanium oxide (TiO₂) and/or strontium titanate (SrTiO₃ or: STO).

Certain objects and advantages have been described herein above. Of course, it is to be understood that not necessarily all such objects or advantages may be achieved in accordance with any particular embodiment of the disclosure. Thus, for example. those skilled in the art will recognize that the disclosure may be embodied or carried out in a manner that achieves or optimizes one advantage or group of advantages as taught herein without necessarily achieving other objects or advantages as may be taught or suggested herein. Further, it is understood that this summary is merely an example and is not intended to limit the scope of the disclosure. The disclosure, both as to organization and method of operation, together with features and advantages thereof, may best be understood by reference to the following detailed description when read in conjunction with the accompanying drawings.

### Brief description of the drawings

FIG.1 is a schematic cross section of a first device according to one embodiment.
FIG.2 and FIG. 3 are diagrams of an energy-band structure to explain operation of the first device.
FIG. 4 is a schematic cross section of a second device according to one embodiment.
FIG. 5 and FIG. 6 are diagrams of an energy-band structure to explain operation of the second device.

In the different drawings, the same reference signs refer to the same or analogous elements.

### Detailed description of certain illustrative embodiments

In the following detailed description, numerous specific details are set forth in order to provide a thorough understanding of the disclosure and how it may be practiced in particular embodiments. However, it will be understood that the present disclosure may be practiced without these specific details. In other instances, well-known methods, procedures and techniques have not been described in detail, so as not to obscure the present disclosure. While the present disclosure will be described with respect to particular embodiments and with reference to certain drawings, the disclosure is not limited hereto. The drawings included and described herein are schematic and are not limiting the scope of the disclosure. It is also noted that in the drawings, the size of some elements may be exaggerated and, therefore, not drawn to scale for illustrative purposes.

Furthermore, the terms first, second, third and the like in the description, are used for distinguishing between similar elements and not necessarily for describing a sequence, either temporally, spatially, in ranking or in any other manner. It is to be understood that the terms so used are interchangeable under appropriate circumstances and that the embodiments of the disclosure described herein are capable of operation in other sequences than described or illustrated herein.

Moreover, the terms top, bottom, over, under and the like in the description are used for descriptive purposes and not necessarily for describing relative positions. It is to be understood that the terms so used are interchangeable under appropriate circumstances and that the embodiments of the disclosure described herein are capable of operation in other orientations than described or illustrated herein.

It is to be noticed that the term "comprising" should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B.

One embodiment relates to solid-state gas sensors for sensing non-redox gases wherein the sensors can operate at ambient temperature, e.g. room temperature. It is an advantage that the need for heating the sensors during their operation can be avoided and that their power consumption can be low.

The concept "gas sensor" is meant to refer to a sensor configured for exposure to a gaseous or liquid environment, into which molecules of a specific chemical compound are dispersed to which the sensor is sensitive.

A gas sensor sensitive to the presence of a specific gas according to one embodiment comprises a first segment, preferably first layer, made of a dielectric material, and a second segment, preferably second layer, made of a semiconducting material. The first segment has a first surface that is exposed to an environment of the gas sensor and a second surface oriented towards the second segment. The first segment is located between the environment and the second segment. The first segment has a first thickness, measured between the first surface and the second surface. The second segment has a second thickness, the second thickness being in the order of the Debye length of the semiconducting material or smaller than the Debye length of the semiconducting material. The first thickness is selected such that upon diffusion of a gas molecule of the specific gas into the first segment, a dipole of the molecule of the specific gas can detectably influence a bending of an energy-band structure of the semiconducting material of the second segment.

In one embodiment, a gas sensor may further comprise a first electrode in electrical contact with the second segment at a first end of the second segment and a second electrode in electrical contact with the second segment at a second end of the second segment. The second segment forms a conduction channel for an electric current, preferably between the first and the second electrode. The bending of the energy-band structure of the semiconducting material of the second segment upon diffusion of a gas molecule of the specific gas into the first segment can be detected by measuring an electrical current through the conduction channel upon applying a voltage difference between the first electrode and the second electrode.

One embodiment is based on the following insights.

Any solid, insulating, semiconducting or conducting has an energy-band structure. The energy-band structure determines the electrical properties, e.g., electrical conductivity, of the solid. The energy-band structure can be bent at the boundaries of the solid. The bending of the energy-band structure at the boundaries usually does not affect the macroscopic electrical properties of the solid. However, if the solid is a semiconductor and has a dimension in the order of the Debye length, the electrical properties are largely determined by the bending of the energy-band structure. The energy-band structure can also be affected by means of an electric field.

Consider a device according to one embodiment, comprising a second segment of semiconducting material with the second thickness in the order of the Debye length of the semiconducting material. The first segment of the dielectric material is deposited over the second segment. The deposition of the dielectric material is typically accompanied by the trapping of electrical charges at the interface of the first segment and the second segment, and in the bulk of the first segment. These trapped charges carry an electric field that generates electrical mirror charges in the semiconducting material, thus affecting the bending of the energy-band structure of the semiconducting material.

Assume now that a molecule of a non-redox gas, e.g., carbon dioxide, is present at the first surface of the first segment or that has diffused into the dielectric material of the first segment via the first surface. The molecule has an electric dipole moment that carries an electric field along. This causes a change in the net charges in the dielectric layer and thus induces a change in the band bending of the semiconductor layer.

The thickness of the dielectric layer (first segment) is preferably tuned to obtain an optimum band-bending in the semiconducting layer. The net charge in the dielectric layer increases with the thickness of the dielectric layer. Therefore, if the thickness of the dielectric layer is too large, the charges present in the dielectric layer may result in a full depletion of the thin semiconducting layer (second segment). This is preferably avoided, because small changes in the charge distribution in the dielectric layer may not detectably influence band-bending (i.e. the semiconducting layer may remain fully depleted). On the other hand, in order to obtain a detectable band-bending in the semiconductor layer, a sufficiently thick dielectric layer is needed. Ultra-thin dielectric layers are not suitable because they may rather passivate the semiconductor surface and keep the energy-bands straight and stable. Therefore, the thickness of the dielectric layer is preferably sufficiently thick to induce noticeable band-bending in the semiconductor layer and sufficiently thin such that changes in dielectric charges due to absorbed gas molecules noticeably affect band-bending in the semiconductor layer. A suitable thickness of the first segment is in the range between about 0.5 nm and 10 nm, e.g. about between 1 nm and 7 nm, for example about 5 nm.

The diffusion length of the molecule of the non-redox gas characterizes the typical path length of the molecule diffusing into the dielectric material and/or diffusing out of the dielectric material. The diffusion length influences the sensitivity of a gas sensor according to one embodiment. It can also influence the recovery rate of a gas sensor of one embodiment. Recovery or refreshing of the gas sensor can be done by flowing an inert gas over the sensor surface. This diffusion length depends heavily on the porosity of the dielectric material. The porosity of the dielectric material can be selected in view of obtaining a predetermined selectivity or a predetermined sensitivity. The diffusion length in the dielectric materials (e.g. atomic-layer-deposited lanthanum-oxide (La₂O₃)) used in test embodiments of the gas sensor according to one embodiment has been estimated as having an upper limit of about 10 nm. The first thickness of the first segment of the dielectric material in the test embodiments was in the order of about 5 nm, which is well below the typical diffusion length of the molecule of the non-redox gas at room temperature. The first thickness of the first segment of the dielectric material in the gas sensor is selected so as to enable gas molecules to diffuse into the dielectric material and to affect the band-bending of the semiconductor material of the second segment through an electric interaction with the trapped charges in the dielectric material of the first segment. The first thickness of the first segment of the dielectric material is preferably not too large so as to avoid that the electric charges in the dielectric material completely deplete an electrically conducting channel in the semiconducting material of the second segment.

When a dielectric layer is deposited over a semiconducting substrate (e.g., a silicon wafer), trapped charges are present in the dielectric layer, such as trapped electrons, trapped holes, mobile ions, immobile ions, charged vacancies (e.g., oxygen vacancies), free -OH radicals or loosely bound -OH radicals, etc. These trapped charges arise from imperfections during the deposition of the dielectric layer. When such a dielectric layer is deposited on a semiconducting substrate, mirror charges corresponding to the charges trapped in the dielectric layer, are induced in the semiconductor near the interface between the semiconductor and the dielectric layer. The electric field of these mirror charges causes either a reduction or an increase in the energy of the conduction band level of the semiconducting substrate, due to the downward band-bending or the upward band-bending in dependence on the type of the local net charge in the dielectric film.

In one embodiment, the bent character of the energy-band structure is used as a basis for implementing a sensor for sensing the presence of a foreign molecule in the environment of the sensor. The foreign molecule diffuses into the dielectric material. While present in the dielectric material, the foreign molecule affects the distribution of the trapped charges and, therefore, the distribution of the charges induced in the semiconducting layer. The changes in the distribution of the induced charges affect, in turn, the band-bending of the energy-band structure of the semiconducting layer and therefore, the electric properties of the semiconducting layer. This is further explained below with reference to the drawings.

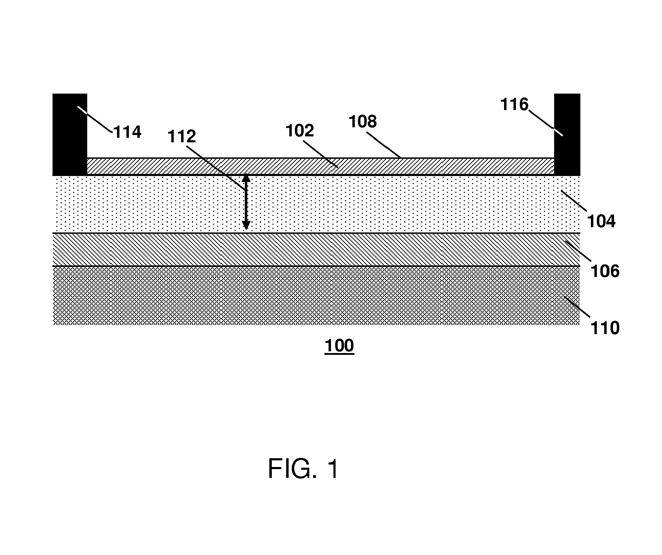

FIG. 1 is a schematic cross section of a first device 100 comprising a gas sensor. The first device 100 comprises a first segment 102 made of a dielectric material, a second segment 104 made of a semiconducting material, and a third segment 106 made of a dielectric material. The dielectric material of the first segment 102 may, but need not, be different (e.g., chemically different) from the dielectric material of the third segment 106. The second segment 104 is sandwiched between the first segment 102 and the third segment 106. The first segment 102 has a first surface 108 that is exposed to an environment of the first device 100. The first segment 102, the second segment 104 and the third segment 106 may be supported by a substrate 110. In the embodiment illustrated in FIG. 1, the first segment 102, the second segment 104 and the third segment 106 form a layered configuration (a stack). The first segment 102, the second segment 104 and the third segment 106 are therefore referred to below as the first layer 102, the second layer 104 and the third layer 106 when discussing the configuration of the first device 100.

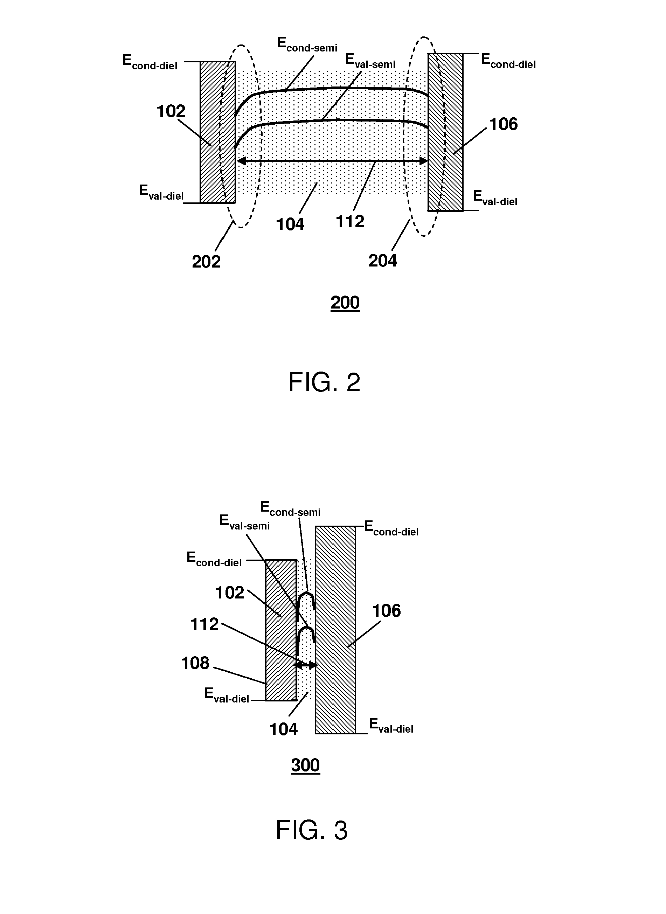

FIG. 2 is a diagram 200 of an energy-band structure of the second layer 104 made of the semiconducting material, the second layer 104 being sandwiched between the first layer 102 and the third layer 106 that are both made of a dielectric material, as in the first device 100 illustrated in FIG. 1. The diagram 200 shows the energy levels of the bottom of the conduction band and of the top of the valence band of the first layer 102, the second layer 104 and the third layer 106. At a first region of contact 202 between the first layer 102 and the second layer 104, and at a second region of contact 204 between the second layer 104 and the third layer 106, the energy-band structure of the second layer 104 is bent. The bending of the energy-band structure affects the electrically conductive properties of the second layer 104 in the first region of contact 202 and in the second region of contact 204. The spatial extent of the band-bending is proportional to the so-called Debye-length of the semiconducting material of the second layer 104. As known, the Debye length is the scale over which mobile electrons or mobile holes screen out electric fields in an electrically conducting or semiconducting medium and over which significant charge separation can occur. The Debye length λD of a material with relative permittivity εr, a density N of the electrical-charge carriers that each carry an electrical charge q, and kept at a temperature T, is given by the mathematical expression λ_{D} = [(ε₀ · εᵣ · k_{B} · T)/ (2 · q² · N)]1/2, wherein k_{B} is Boltzmann's constant and ε0 is the vacuum permittivity. For doped silicon, the relative permittivity εᵣ is about 11.75. At room temperature, the Debye length is about 24 micrometer for intrinsic silicon with a mobile carrier concentration of about 1.45×10¹⁰ cm⁻³. However, the resistivity of intrinsic silicon is too high for a sensing device. The resistivity and the Debye length decrease by doping. For a doping level of 10¹⁵ cm⁻³, the Debye length is about 91 nm at room temperature. Therefore, if the second layer 104 is made of doped silicon and the thickness 112 is approximately 100 nm, the electrical properties of the second layer 104, e.g., the electrical conductivity, are largely determined by the band-bending.

Consider now a thickness 112 of the second layer 104, i.e., a minimum distance between the first layer 102 and the third layer 106, measured across the second layer 104. If the thickness 112 is much larger than the Debye length of the semiconducting material of the second layer 104 (illustrated in FIG. 2), the bending of the energy bands does not substantially affect the electrical properties, e.g., the electrical conductivity, of the second layer 104.

FIG. 3 is a diagram 300 of the energy-band structure of the second layer 104 sandwiched between the first layer 102 and the third layer 106, wherein the thickness 112 of the second layer 104 is in the order of the Debye length of the semiconducting material of the second layer 104. The electrical properties of the second layer 104 are now largely determined by the band bending. This is illustrated in FIG. 3, wherein the bottom of the conduction band of the semiconducting material of the second layer 104 and the top of the valence band of the semiconducting material of the second layer 104 have shapes that substantially deviate from a substantially flat shape.

In one embodiment, the sensing mechanism of a gas sensor is based on changing the charge distribution in the first layer 102 of dielectric material as a result of exposing the first surface 108 of the first layer 102 to an analyte fluid (a gas or a liquid). For example, the analyte fluid can comprise molecules of a non-redox substance, e.g., carbon-dioxide CO₂. If a molecule of such non-redox substance diffuses into the first layer 102, the electric field of the electric dipole of the molecule interacts with charges present in the dielectric material of the first layer 102. This interaction changes the electric field of the trapped charges. As a result, the mirror charges, induced in the semiconducting material of the second layer 104 by the trapped charges, change as well and cause the energy-band structure of the semiconducting material of the second layer 104 to change.

The net charge density in the first layer 102 of dielectric material increases with the thickness of the first layer 102. Therefore, an ultra-thin first layer 102 of dielectric material is preferably avoided in a gas sensor in one embodiment. If the first layer 102 were to be ultra-thin, the first layer 102 would rather passivate the surface of the second layer 104 of semiconducting material semiconductor, and maintain a stable energy-band structure in the semiconducting material. In order to obtain a detectable band bending, the first layer 102 of dielectric material needs a sufficient thickness so as to have an adequate density of trapped charges. The thickness of the first layer 102 of dielectric material therefore is preferably selected to cause a sufficient band-bending in the semiconducting material of the second layer 104. If the second layer 104 of semiconducting material is too thin and the first layer 102 of dielectric material is too thick, the band bending in the semiconducting material may be so strong that a small change in the trapped charges in the dielectric material, due to the absorption of gas molecules, will not noticeably influence the bending of the energy-bands.

In one embodiment, the sensitivity of the gas sensor in a device could be increased by using a dielectric material in the first layer 102 that has a high electric susceptibility _{Xe}. The electric susceptibility _{Xe} relates to the dielectric permittivity εᵣ according to the (tensor-) relationship Xₑ = εᵣ -1, and specifies the vector of polarization P according to P = ε₀ XeE, wherein the vector E stands for the external electric field. A dielectric material of a high electric susceptibility _{Xe} can be easily polarized by the dipole of the molecule in the analyte fluid and as a result transfer the electric field of the dipole to the semiconducting material of the second layer 104.

Any change in band-bending results in a change in the electrical conduction through the second layer 104, which can be measured, e.g., electrically. For example, and with reference to FIG. 1, the first device 100 comprises a first electrode 114 that contacts the second layer 104 at one end, and a second electrode 116 that contacts the second layer 104 at another end. The second layer 104 of semiconducting material provides an electrically conductive channel between the first electrode 114 and the second electrode 116. The first device 100 has a configuration similar to a field-effect transistor (FET) configuration, i.e. a FET-like structure (without a gate electrode). Accordingly, the first device 100 is configured for the detection of non-redox gases and uses a thin first layer 102 of a dielectric material (with a thickness in a range of sub-nanometer to about 10 nm, e.g. about 5 nm), which is deposited over a channel area of a FET-like structure. The first layer 102 contains a large number of defects and vacancies. This first layer 102 of dielectric material, being thin, can also be used as a protective film for protecting the first device 100 against undesirable influences from the environment. The dielectric material of the first layer 102 preferably has an adequate thickness so as to have a sufficient but not too high density of trapped charges. If the first layer 102 of dielectric material has a thickness of about 5 nm, the first layer 102 functions adequately for sensing as well as for protecting.

The first device 100 can be manufactured in a variety of manners.

The second layer 104 of a semiconducting material can be deposited as a thin film on the third layer 106 of an insulating material. The semiconducting material of the second layer 104 can for example comprise silicon (Si), indium arsenide (InAs), indium phosphide (InP), gallium arsenide (GaAs), zinc oxide (ZnO₂), tungsten trioxide (WO₃) tin dioxide (SnO₂), etc. These semiconducting materials can be used as is, or can be used with dopants to increase the free carrier density and, therefore, the electrical conductivity. The electrically insulating third layer 106 comprises a dielectric material such as, e.g., a thick silicon oxide (SiOx) or silicon nitride (Si₃N₄). The third layer 106 has been deposited on the substrate 110, e.g., a silicon wafer. Next, the second layer 104 is patterned using for example a photolithographic technique and the Ohmic contacts are formed to be used as the first electrode 114 and the second electrode 116 later on. The first layer 102 of dielectric material can be deposited before or after forming the first electrode 114 and the second electrode 116.

It may be preferred to create the thin second layer 104 of semiconducting material in a silicon-on-insulator (SOl) technology, as this SOl technology enables efficient charge depletion throughout the volume of the second layer 104.

The thin second layer 104 of semiconducting material may be crystalline or amorphous. If the second layer 104 is made of (doped) silicon, and the third layer 106 is made of silicon dioxide, the crystallinity and the resistivity of the second layer 104 depend on the fabrication process. For example, plasma-enhanced chemical vapor deposition (PECVD) of the second layer 104 results in an amorphous silicon film. As another example, low-pressure chemical vapor deposition (LPCVD) of silicon results in polycrystalline silicon that can be doped afterwards e.g. by means of implantation to form the second layer 104. SOI, on the other hand, gives rise to a monocrystalline silicon layer 104.

As an alternative to the above process of forming the second layer 104, one or more nanowires of a semiconducting material can be deposited or grown on an insulating layer, e.g., the third layer 106, or on an insulating substrate, e.g., the substrate 110. The one or more nanowires may be formed horizontally with respect to the substrate 100, i.e. with their longitudinal direction substantially parallel to the substrate surface. The one or more semiconducting nanowires are then used as an electrically conductive channel in a gas sensor in one embodiment.

In a fabrication method, the first layer 102 of dielectric material can be deposited over the second layer 104 of the semiconducting material and over the Ohmic contacts, e.g. by means of atomic layer deposition (ALD) or chemical vapor deposition (CVD). ALD or CVD can be very efficiently used to deposit the thin first layer 102 of dielectric material. Examples of such a dielectric material are lanthanum oxide (La₂O₃), titanium oxide (TiO₂), strontium titanate (SrTiO₃ or: STO), etc. The deposition conditions of the first layer 102 of dielectric material in ALD or CVD can be adjusted to tune the charge density in the first layer 102.

In case the second layer 104 is formed by one or more horizontal nanowires of semiconducting material, once the horizontal nanowires have been grown or deposited, the first layer 102 of ultra-thin dielectric material can be deposited on the one or more nanowires by means of ALD or CVD with good step-coverage. The first layer 102 of dielectric material can be deposited before or after the one or more nanowires are electrically contacted with the first electrode 114 and the second electrode 116.

In certain embodiments, the conditions that determine the bending of the energy bands in the second layer 104 can be controlled using an electric field. The first device 100 can be fabricated in a FET-like configuration, wherein the second layer 104 functions as a current conduction channel. The control of band-bending in the second layer 104 can be achieved by using a back-gate configuration (not shown) in the first device 100.

The second layer 104 of semiconducting material provides an electrically conductive channel between the first electrode 114 and the second electrode 116. The change in conductivity of the channel can be measured, typically by means of applying a DC bias (voltage difference) over the channel (i.e. between first electrode 114 and second electrode 116). One could use a configuration, wherein in operation an electrical current is flowing through the channel in the absence of the specific gas to be detected. The presence of the gas molecules is then measured as a change in the electrical current through the channel. Alternatively, one could use a power-saving configuration. In the power-saving configuration, the energy bands of the semiconducting material of the second layer 104 are configured to be bent such that the channel is normally blocked, i.e. such that substantially no current is flowing through the channel in the absence of a target gas or analyte fluid. Upon diffusion of the gas molecules of the analyte fluid into the dielectric material of the first layer, the bending of the energy-bands is reduced so as to render the channel electrically conductive. The bending of the energy-bands in the non-conducting state of the channel is tuned so as to have a less strong bending in the presence of the gas molecules. This tuning depends on, e.g., the type of electrical charges trapped in the dielectric material of the first layer 102, the first thickness of the first layer 102 of dielectric material and the second thickness of the second layer 104 of the semiconducting material. It can also be influenced by applying a control voltage on a back gate in a back-gate configuration.

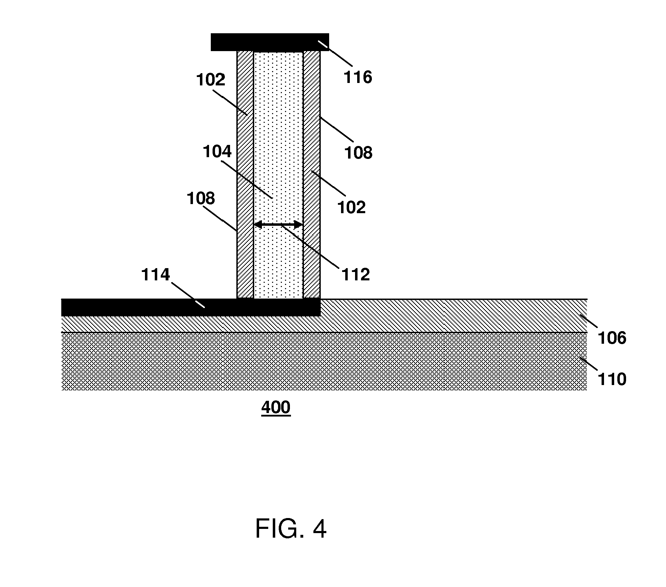

FIG. 4 is a schematic cross section of a second device 400 comprising a gas sensor according to one embodiment. The second segment 104 in the second device 400 is now formed as one or more nanowires of semiconducting material in a vertical orientation relative to the horizontal substrate 110. FIG. 4 shows the second segment 104 as formed by a single nanowire by way of example. The nanowire of the second segment 104 is coated or otherwise enclosed by the first segment 102 of a dielectric material as a thin film.

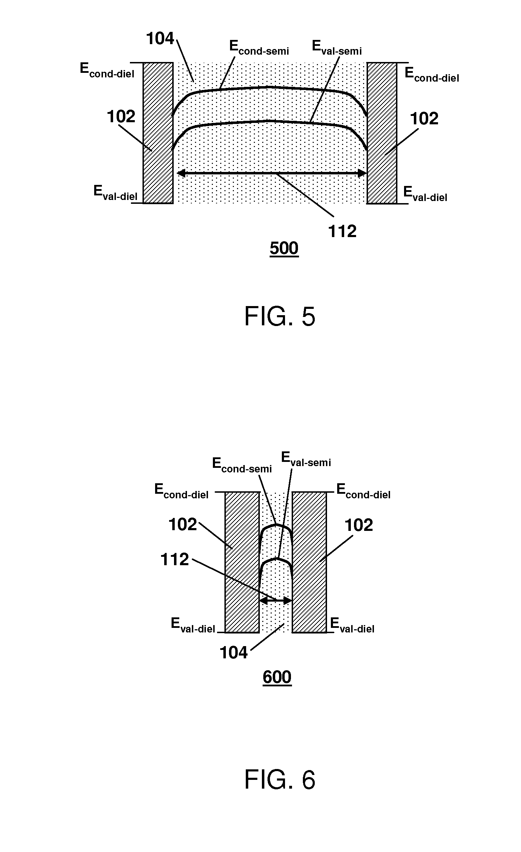

FIG. 5 is a diagram 500 of an energy-band structure of the second segment 104 made of the semiconducting material, and coated with the first segment 102 of the dielectric material over its length. FIG. 6 is a diagram 600 of the energy-band structure of the second segment 104 in case the second segment is made in the form of a nanowire. The diagram 600 shows that the energy-band structure is bent all over the thickness of the nanowire and, therefore, determines the electrical properties of the semiconducting material of the nanowire.

The foregoing description details certain embodiments of the disclosure. It will be appreciated, however, that no matter how detailed the foregoing appears in text, the disclosure may be practiced in many ways. It should be noted that the use of particular terminology when describing certain features or aspects of the disclosure should not be taken to imply that the terminology is being re-defined herein to be restricted to including any specific characteristics of the features or aspects of the disclosure with which that terminology is associated.

While the above detailed description has shown, described, and pointed out novel features of the disclosure as applied to various embodiments, it will be understood that various omissions, substitutions, and changes in the form and details of the device or process illustrated may be made by those skilled in the technology without departing from the spirit of the disclosure.

## Claims

1. A device (100) comprising a gas sensor sensitive to the presence of a specific gas, the gas sensor comprising: a first segment (102) made of a dielectric material; and a second segment (104) made of a semiconducting material; wherein: the first segment (102) has a first surface (108) that is exposed to an environment of the gas sensor; the first segment (102) is located between the environment and the second segment (104); the first segment (102) has a first thickness; the second segment (104) has a second thickness (112); wherein the first thickness is selected such that upon diffusion of a gas molecule of the specific gas into the first segment (102), a dipole of the molecule of the specific gas detectably influences a bending of an energy-band structure of the semiconducting material of the second segment (104); and wherein the second thickness is in the order of the Debye length of the semiconducting material or smaller than the Debye length of the semiconducting material.

2. A device (100) according to claim 1, wherein the device (100) further comprises a first electrode (114) in electrical contact with the second segment (104) and a second electrode (116) in electrical contact with the second segment (104), and wherein the second segment (104) forms a conduction channel for an electrical current between the first electrode and the second electrode.

3. A device (100) according to claim 1 or 2, wherein: the gas sensor comprises a third segment (106) of a further dielectric material; and the second segment (104) is sandwiched between the first segment (102) and the third segment (106).

4. A device (100) according to any one of claims 1 - 3, wherein the gas sensor comprises a back-gate for receiving a control voltage.

5. A device (100) according to any one of claims 1 - 4, wherein the second segment (104) has a length substantially larger than the second thickness and wherein the first segment (102) encloses the second segment (104) over the length of the second segment.

6. A device (100) according to any one of claims 1 -5, wherein the second segment (104) is formed as at least one nanowire.

7. A device (100) according to any one of claims 1 -6, wherein the first thickness is in the range between about 0.5 nm and 10 nm, e.g. about between 1 nm and 7 nm, for example about 5 nm.

8. A device (100) according to any one of claims 1- 7, wherein the semiconducting material of the second segment (104) comprises silicon (Si), indium arsenide (InAs), indium phosphide (InP), gallium arsenide (GaAs), zinc oxide (ZnO₂), tungsten trioxide (WO₃) and/or tin dioxide (SnO₂).

9. A device (100) according to any one of the claims 1 -8, wherein the dielectric material of the first segment (102) comprises lanthanum oxide (La₂O₃), titanium oxide (TiO₂) and/or strontium titanate (SrTiO₃ or: STO).

10. Method of manufacturing a gas sensor sensitive to the presence of a specific gas for use in the device (100) according to any one of claims 1 - 9, the gas sensor comprising a first segment (102) and a second segment(104) , the first segment (102) having a first surface provided to be exposed to an environment of the gas sensor and a second surface oriented towards the second segment, the first segment (102) provided for being located between the environment and the second segment (104), the first segment (102) having a first thickness, measured between the first surface and the second surface, the second segment (104) having a second thickness, the second thickness being in the order of the Debye length of the semiconducting material or smaller than the Debye length of the semiconducting material, the first thickness being selected such that upon diffusion of a gas molecule of the specific gas into the first segment (102), a dipole of the molecule of the specific gas can detectably influence a bending of an energy-band structure of the semiconducting material of the second segment (104).

11. Method of manufacturing a gas sensor according to claim 10, wherein the method comprises forming the first segment (102) by depositing the dielectric material on the second segment.

12. Method of manufacturing a gas sensor according to claim 11, wherein the depositing preferably comprises one of: an atomic layer deposition (ALD) method and a chemical vapor deposition (CVD) method.

13. Use of the device (100) according to any one of claims 1 -9 for sensing the presence of a specific gas in the environment of the gas sensor, wherein the first surface of the first segment (102) is exposed to the environment of the gas sensor; wherein upon diffusion of a gas molecule of the specific gas into the first segment (102), a dipole of the molecule of the specific gas detectably influences a bending of the energy-band structure of the semiconducting material of the second segment (104).

14. Use according to claim 13, wherein the specific gas is a non-redox gas.

15. Use according to claim 13 or 14 in combination witch claim 2, wherein the bending of the energy-band structure of the semiconductor material of the second segment (104) is detected by measuring a difference in an electrical current through the conduction channel upon applying a voltage difference between the first electrode and the second electrode.
